# EUROPEAN PATENT APPLICATION

(11) **EP 1 044 618 A1**
(43) Date of publication of application: **18.10.2000**
(21) Application number: 99830217.8
(22) Date of filing: 15.04.1999
(51) Int. Cl.: A43B 7/26, A61F 5/01

(54) **Arch support with adjustable strap for the correction of hallux valgus**

(71) Applicant: Astra S.A.S. di Marcocci Giuseppe & C., San Venanzo (TR) (IT)
(72) Inventor: Marcocci, Giuseppe, San Venanzo (TR) (IT)
(74) Representative: Baldi, Claudio

(57) **Abstract**

The present invention relates to an arch support for shoes for the correction of the hallux valgus, provided with a leather strap (2) in the front part which supports the toes, placed in orthogonal position with respect to the longitudinal axis of the arch support, used to embrace and hold the user's hallux valgus; it being provided that the free end of the strap can be turned down and fixed under the arch support.

## Description

The present patent application relates to an arch support for shoes provided with ad adjustable strap used to hold the hallux valgus in its natural anatomical position. As it is known, the hallux valgus causes very high pain. As a matter of fact, a considerable amount of pain is a temporary consequence of the surgical operation carried out to eliminate the disease, as well.

The hallux valgus consists in the deviation of the big toe towards the other toes, resulting in the painful swelling at the base of the big toe, accompanied by the anatomical disarrangement of the entire forefoot. The more acute the hallux valgus is, the lower is the capacity of the foot in terms of support and push.

So, the foot starts reacting to the unequal distribution of the body weight, first through the thickening of the forefoot, by creating a kind of natural sole, and then by increasing the support of the fingertips of the other toes, which turn into hooked or hammertoes.

When the knuckles of the toes keep hitting the upper of the shoe, the pain becomes unbearable and the surgical operation for the resection of the bone tumescence becomes necessary.

As a matter of fact, the acute phase of the disease forces the patient to avoid the use of shoes with upper, not only shoes for outdoor use, but also slippers. In other words, in this case the patient is forced to wear sandals with no upper.

In this pathological situation, the arch support according to the invention proves extremely effective, since it is provided with a special strap used to embrace the hallux valgus to hold it (and bring it back, if needed) in its natural correct position, that is on the same axis as the other toes, without surmounting the adjacent toe.

In this perspective it is easy to understand how the use of the arch support according to the present invention inside the shoe allows the subjects who suffer from hallux valgus to wear any type of shoes or slippers with no discomfort.

For major clarity the description of the invention continues with reference to the enclosed drawing, which is intended for purposes of illustration and not in a limiting sense, in which:
- figure 1 is an axonometric view of the arch support according to the invention with the protection strap in non operational condition;
- figure 2 is a view of the lower surface of the arch support, which shows how the strap is fixed under the arch support.

With reference to the above figures, the device according to the invention consists in an arch support (1) provided with a leather strap (2) in the front part which supports the toes, fixed at a short distance from the internal edge (1a) of the arch support (1) and placed in orthogonal position with respect to the longitudinal axis of the arch support.

In particular, the distance between the fixing point of the strap (2) and the edge (1a) of the arch support must allow for introducing the big toe and placing it on the arch support (1) in an easy, comfortable way.

A short piece of elasticized band (2a) and next to it a piece of velcro (2b) are applied at the free end of the leather strap (2).

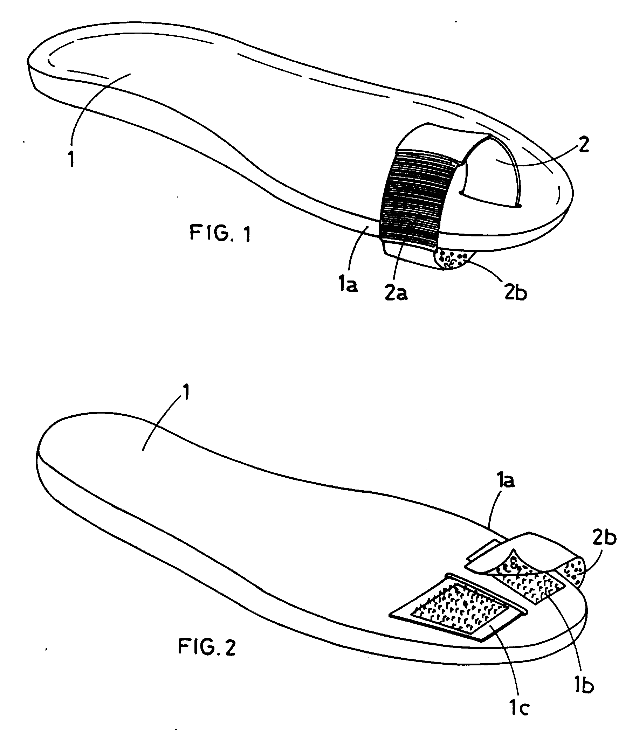

The velcro piece (2b) is hooked to one of the two velcro inserts (1b and 1c), applied under the arch support (1), when the strap (2) embraces the hallux valgus and turns down under the arch support (1), as shown in figure 2.

Obviously, the presence of the elasticized band (2a), together with the possibility of fixing the free velcro end (2b) of the strap (2) to one of the velcro inserts (1b and 1c) located under the arch support (1) allows to adjust the tension of the strap (2) and to reduce or increase the cross-section of the eyelet formed with the strap (2) as needed.

This allows for adjusting the force used by the strap (2) to bring back or hold the big toe of the user in its correct anatomical position, as necessary.

It is understood that, without exiting the scope of the present invention, a single velcro insert of considerable length in transversal direction may be provided under the arch support (1) in replacement of the parallel pair of short inserts (1b and 1c) shown in figure 2.

Likewise, different solutions can be adopted to fix the free end of the strap (2) under the arch support (1).

According to a first solution, the end of the strap (2) is provided with buttons capable of being placed and fitted into suitable buttonholes located under the arch support (1). Alternatively, the free end of the strap (2) can be fitted and hooked to a suitable buckle, preferably housed in an appropriate compartment created under the arch support (1).

## Claims

1. Arch support for shoes, provided with a leather strap (2) in the front part which supports the toes, fixed at a short distance from the internal edge (1a) of the arch support (1) and placed in orthogonal position with respect to the longitudinal axis of the arch support; it being provided that the strap (2), which features a short piece of elasticized band (2a) near its free end, can be turned down and hooked under the arch support (1).

2. Arch support for shoes, according to claim 1, characterized in that the free end of the strap (2) is provided with a piece of velcro (2b) that can be hooked under the arch support (1) to one or more velcro inserts located therein.

3. Arch support for shoes, according to claim 1, characterized in that the free end of the strap (2) is provided with buttons capable of being placed and fitted into suitable buttonholes located under the arch support (1).

4. Arch support for shoes, according to claim 1, characterized in that the free end of the strap (2) is capable of being fitted and hooked to a suitable buckle, preferably housed in a appropriate lowered compartment located under the arch support (1).
